# EUROPÄISCHE PATENTSCHRIFT

(11) **EP 1 902 691 B1**
(45) Veröffentlichungstag und Bekanntmachung des Hinweises auf die Patenterteilung: **15.04.2009**
(21) Anmeldenummer: 07002866.7
(22) Anmeldetag: 10.02.2007
(51) Int. Cl.: A61F 5/02

(54) **Osteoporose-Orthese**
Osteoporosis orthosis
Orthèse pour ostéoporose

(30) Priorität: 23.09.2006 DE 202006014659 U
(43) Veröffentlichungstag der Anmeldung: 26.03.2008
(73) Patentinhaber: Karl Werkmeister, Medizinische Leibbinden, 37281 Wanfried (DE)
(72) Erfinder: Germerodt, Hans-Jürgen, 37281 Wanfried (DE)
(74) Vertreter: Walther, Walther & Hinz GbR

(56) Entgegenhaltungen:
- DE-A1- 2 027 722
- DE-A1- 4 117 768
- DE-A1- 10 329 454
- DE-A1- 19 603 173
- DE-U1- 20 009 763
- DE-U1- 29 720 475
- US-A1- 2005 181 705

## Beschreibung

Die Erfindung betrifft eine Osteoporose-Orthese, umfassend eine Pelotte und ein Mieder mit einer Rückentasche zur Aufnahme der Pelotte,
wobei die Pelotte um ihre Längsachse tordierbar ist.

Osteoporose befällt als Krankheit im Wesentlichen Frauen.

Mit der Osteoporose einhergehend findet eine Buckelbildung im Rückbereich statt. Einer solchen Buckelbildung wird nun dadurch versucht entgegen zu wirken, dass man bestrebt ist, die Wirbelsäule der Person und damit die Person selbst im Bereich des Rückens aufrecht zu halten. Durch eine solche Pelotte, die in der Rückentasche eines Mieders angeordnet ist, wird daher versucht, Osteoporose-Patienten dazu zu bewegen, den Rücken aufgerichtet zu halten. Dies geschieht im Einzelnen dadurch, dass die Pelotte, die in Längsrichtung relativ steif ausgebildet ist, und aufgrund ihrer Form und ihrer Verbindung mit dem Mieder das Bestreben hat, ihre Form beizuhalten. Verfällt nun der Patient in eine "Buckelstellung", dann ist das Mieder mit der Pelotte bestrebt, den Menschen bzw. den Rücken wieder aufzurichten. Man spricht insofern auch von einer sogenannten Mahn-Pelotte. Das heißt, solche Pelotten bewirken eine Korrektur der Rückenstellung der Patienten durch Mahnung an den Patienten, die korrekte Stellung einzunehmen. Nimmt der Patient die korrekte Stellung ein, ist hiermit einhergehend eine Stärkung der Rückenmuskulatur verbunden, die eine Stärkung des Rückens bewirkt. Ein durch eine entsprechende Ausbildung der Muskulatur gestärkter Rücken wird leichter in gerader Position gehalten, als ein Rücken ohne oder nur mit geringer Muskulatur. Das bedeutet zusammenfassend, dass durch die Pelotte in Verbindung mit dem Mieder der Patient an die Einnahme einer korrekten Stellung erinnert wird, durch die Einnahme der korrekten Stellung die Rückenmuskulatur gestärkt wird und hiermit einhergehend durch die Stärkung der Rückenmuskulatur der Patient nach und nach von allein den Rücken in aufrechter Position halten kann. Insofern findet durch die Orthese auch eine Korrektur der Rückenstellung statt.

Bekannte Osteoporose-Orthesen haben aber den Nachteil, dass die Pelotten nicht nur in Längsrichtung steif ausgebildet sind, was sie durchaus sein sollen, sondern darüber hinaus auch in Querrichtung. Eine erhöhte Steifigkeit einer solchen Pelotte in Querrichtung bedeutet, dass bei Torsion des Oberkörpers relativ zur Hüfte die bekannten Pelotten einer solchen Bewegung entgegenwirkten. Das heißt, die Beweglichkeit des Rückens im Hinblick auf Drehbewegungen ist stark eingeschränkt.

Aus der DE-OS 196 03 173 A1, die den nächsten Stand der Technik darstellt, ist eine Pelotte bekannt, bei der parallel zur Wirbelsäule zu beiden Seiten der Wirbelsäule sogenannte Stützelemente vorgesehen sind, mit deren Hilfe es nunmehr möglich ist, die Krafteinleitung von der Wirbelsäule in die Stützvorrichtung in Wirbelsäulenrichtung zu ermöglichen.

Die DE 41 17 768 A1 zeigt eine Pelotte zum Stützen der menschlichen Wirbelsäule im Bereich der Lendenwirbel. Diese Petotte zeichnet sich auf ihrer dem Rücken zugewandten Seite dadurch aus, dass sie zwei längsverlaufende dornartige Fortsätze aufweist, durch die eine Einbuchtung zwischen diesen Fortsätzen gebildet wird, um sicherzustellen, dass die Wirbelkörper nicht während des Tragens an der Pelotte anliegen.

Aus dem DE 297 20 475 U1 ist eine Pelotte in Form einer abstützenden steifen Schiene bekannt, um z B. nach Wirbelsäulenbrüchen die Wirbelsäule zu stabilisieren. Diese Pelotte soll sich über die gesamte Länge der Wirbelsäule erstrecken, also von den Halswirbeln bis zum Steißbein. Die Beweglichkeit dieser Pelotte ist aufgrund der Aufgabenstellung der Stützung des Wirbelsäulenapparates naturgemäß stark eingeschränkt.

Aus der DE 103 29 454 A1 ist eine, orthopädische Stützeinrichtung für Rücken- und Lumbalbereich in Form einer rucksackartig anzulegenden Rückenorthese bekannt. Diese Orthese umfasst eine Pelotte mit einem Gurtträgersystem, wobei das Gurtträgersystem dafür sorgt, die Pelotte im Bereich des Rückens zu fixieren. In Bezug auf die Pelotte ist in dieser Literaturstelle ausgeführt, dass die Pelotte elastisch ausgebildet ist

Die Wirbelsäule zeigt im Bereich der Lordose des Rückens zum Gesäß hin eine Verformung nach außen. Im Bereich des Halses, also im Nackenbereich der Wirbelsäule, ist die Wirbelsäule nach vorn ausgerichtet, so dass sich die charakteristische Form eines Fragezeichens der Wirbelsäule ergibt.

Grundsätzlich besteht die Aufgabe einer Pelotte darin, dafür zu sorgen, dass die Patientin, die eine solche Pelotte trägt, daran erinnert wird, sich gerade zu halten. Die Patientin soll allerdings im Wesentlichen nicht durch die Pelotte in ihrer Beweglichkeit eingeschränkt werden. Das heißt, es soll nach wie vor möglich sein, ohne dass dies unbequem ist, sich mit dem Oberkörper retativ zum Becken zu drehen oder sich auch zu bücken. Eine solche Orthese, die sich in Form eines Fragezeichens über den gesamten Rücken erstreckt, und zwar vom Brustwirbelbereich bis zum Bereich der Lordose, hemmt insofern die Bewegung des Bückens, da die Form der Pelotte im Bereich der Lordose der Biegung der Wirbelsäule beim Bücken genau entgegengesetzt ist. Das heißt, das Bücken ist mit den bekannten Orthesen unbequem.

Der Erfindung liegt daher die Aufgabe zu Grunde, eine Osteoporose-Orthese der eingangs genannten Art bereitzustellen, die es insbesondere auch ermöglicht, dass die Pelotte einem Bückvorgang nicht entgegenwirkt.

Zur Lösung der Aufgabe ist insofern erfindungsgemäß vorgesehen, dass die Pelotte im Bereich der Lordose einen quer zur Längsachse biegsamen Abschnitt aufweist, wobei der biegsame Abschnitt durch ein von dem Material der Pelotte unterschiedlichem Material gebildet ist.

Im Einzelnen kann hierbei der biegsame Abschnitt aus einem von dem Material der Pelotte unterschiedlichen weicheren oder biegsamen Material gebildet werden. Denkbar ist ein Einsatz, z. B. aus Metall, der beim Kunststoffspritzen der Pelotte mit eingespritzt wird.

Als besonders vorteilhaft, insbesondere in Bezug auf die Haltbarkeit, hat sich herausgestellt, wenn der untere Teil der Pelotte, also der Lordosenabschnitt, durch ein Verbindungsglied aus biegsamem Material, z. B. TPE, verbunden ist. Dieses Verbindungselement weist zu beiden Enden eine U-förmige Ausnehmung zur Aufnahme des freien Endes der Pelotte und des entsprechenden des Lordosenabschnitts auf. Die Kontur des Verbindungselements ist hierbei in etwa der Querschnittsform der Pelotte nachempfunden. Das Verbindungselement kann eingespritzt sein oder als gesondertes Element z. B. durch Kleben mit der Pelotte bzw. dem Lordosenabschnitt der Pelotte verbunden sein. Dieses Verbindungselement weist darüber hinaus einen Mittelabschnitt aus Vollmaterial auf.

Weitere vorteilhafte Merkmale ergeben sich aus den Unteransprüchen.

Um die Pelotte der Anatomie des jeweiligen menschlichen Rückens besser anpassen zu können, ist nach einem weiteren Merkmal der Erfindung ' vorgesehen, dass die Pelotte aus thermoplastischen Material besteht. Denn ein solches thermoplastisches Material ermöglicht die nachträgliche Verformung unter Heißluft zur Anpassung an den jeweiligen Patienten.

Im Einzelnen ist des Weiteren vorgesehen, dass die Pelotte im Querschnitt im Bereich der Wirbelsäule eine sich in Längsrichtung erstreckende Ausbuchtung oder Wölbung aufweist, wobei sich zu beiden Seite der Ausbuchtung jeweils ein Stützrand erstreckt, mit dem die Pelotte auf den Muskelpartien beiderseits der Wirbelsäule aufliegt. Die Ausbuchtung oder Wölbung parallel zur Längsachse der Pelotte bewirkt ähnlich einer Sicke die Versteifung der Pelotte parallel zur Längsachse der Pelotte. Gleichzeitig bewirkt diese Ausbuchtung oder Wölbung allerdings auch, dass die Pelotte nicht auf den Dornfortsätzen der Wirbelsäule aufliegt und die Stützränder zu beiden Seiten der Wölbung eine Torsion der Pelotte um die Wölbung herum ermöglichen. Bevorzugt ist in diesem Zusammenhang vorgesehen, dass der Stützrand zu jeder Seite beabstandet zueinander in Längsrichtung Einschnitte oder Ausnehmungen aufweist. Diese Einschnitte oder Ausnehmungen unterstützen die Tordierbarkeit der Petotte ganz erheblich. Das heißt, der Stützrand, der sich bevorzugt seitlich über die Muskelseitenstränge erstreckt, bewirkt eine relativ hohe Elastizität bei Torsion der Pelotte. Eine Erstreckung des Stützrandes seitlich über die Muskelseitenstränge bedeutet nicht, dass sich der Stützrand notwendigerweise über den Muskelseitenstrang hinaus erstreckt, es bedeutet, dass sich der Stützrand zumindest über die Breite des Muskelseitenstranges erstreckt. Dies deshalb, um Druckstellen im Bereich des Muskelseitenstranges durch Anliegen der Pelotte zu vermeiden. Dem gleichen Ziel dient im Übrigen auch, dass in der Einschubtasche zum Rücken zu ein sogenanntes Abstandsgewirkes eingelegt ist. Dieses Abstandsgewirke ist ein etwas dickerer, sehr luftdurchlässiger Stoff, der im Übrigen die Neigung zum schwitzen in diesem Bereich herabsetzt. Auch die in Querrichtung der Pelotte gewölbte Form der Pelotte verhindert die Bildung von Druckstellen, da diese durch die Wölbung vollflächig am Rücken anliegt.

Der Stützrand ist im Nackenbereich breiter als im Lordosenbereich. Wie bereits zu eingangs erläutert, soll durch die Pelotte in Verbindung mit dem Mieder erreicht werden, dass der Patient im Bereich des oberen Rückens aufgerichtet wird. Das heißt, in diesem Bereich übt die Pelotte eine relativ große Kraft auf den Oberkörper des Patienten aus. Durch einen verbreiterten Stützrand im Nackenbereich wird diesem insofern Rechnung betragen, als durch den verbreiterten Stütztand das Mieder über eine größere Fläche nach hinten gezogen wird.

Betrachtet man in diesem Zusammenhang die Ausbildung des Mieders, so ist vorgesehen, dass das Mieder im oberen Rückenbereich Züge aufweist, die die Rückentasche für die Pelotte kreuzartig queren und die im Brustbereich bis in den Schultersteg geführt sind. Wie bereits ausgeführt, ist die Krafteinwirkung der Pelotte auf den menschlichen Rücken im oberen Rückenbereich am größten. Damit durch das Mieder die entsprechenden von der Pelotte aufgebrachten Kräfte auf den Rücken übertragen werden können, sind die erfindungsgemäßen Kreuzzüge vorgesehen.

Damit das Mieder in Verbindung mit der Pelotte die entsprechenden Kräfte im oberen Bereich des Rückens aufbringen kann, ist erforderlich, dass die Pelotte der im Bereich der Lordose an dem Rücken relativ fest anliegt. Insofern weist das Mieder im Bereich der Lordose einen quer zur Längsachse der Wirbelsäule verlaufenden Querzug/Unterstützungsgurt auf, wobei dieser Querzug unmittelbar oberhalb des biegsamen Abschnittes über die Einschubtasche für die Pelotte geführt ist. Dieser Querzug ist insbesondere lösbar, z. B. mittels eines Klettverschlusses auf der Vorderseite des Mieders fixierbar, so dass durch diesen Querzug die Möglichkeit besteht, die Pelotte eng an die Wirbelsäule anzupressen. Dies bedeutet, dass je fester die Pelotte im Bereich der Lordose an dem Rücken des Patienten anliegt, um so höher ist die Kraft, die schlussendlich von der Pelotte über das Mieder im oberen Bereich des Rückens auf den Rücken des Patienten ausgeübt wird.

Dem Komfort dient ebenfalls, dass das Mieder auf der Vorderseite einen beidseitig öffenbaren Reißverschluss aufweist, so dass das Mieder bei wiederkehrenden täglichen Vorrichtungen nicht immer vollständig geöffnet werden muss.

Anhand der Zeichnungen wird die Erfindung nachstehend beispielhaft näher erläutert.
- Fig. 1: zeigt die Pelotte in perspektivischer Darstellung;
- Fig. 2: zeigt das Mieder in einer Ansicht von vorn;

- Fig. 3: zeigt das Mieder in einer Ansicht von hinten;
- Fig. 4: zeigt die Pelotte mit einem Verbindungselement in perspektivischer Darstellung;
- Fig. 5: zeigt das Verbindungselement mit den Enden der Pelotte bzw. dem Lordosenabschnitt in einer Seitenansicht.

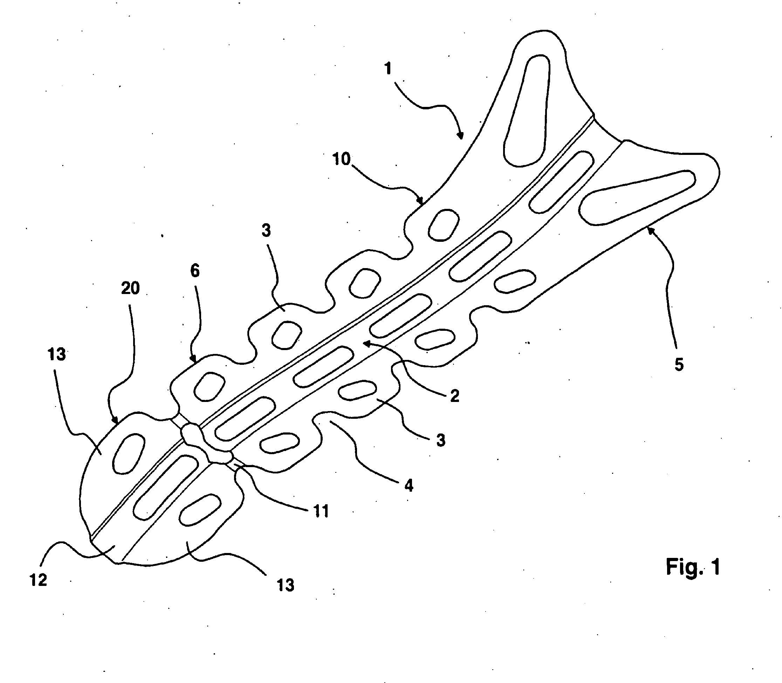

Die Ausbildung der Pelotte ergibt sich in Anschauung von Fig. 1; die mit 1 bezeichnete Pelotte unterteilt sich in zwei Abschnitten, nämlich einen Rückenbereich 10 und einen Lordosen- oder Lendenwirbelabschnitt 20. Der Rückenbereich 10 ist mit dem Lendenwirbel- oder Lordosenabschnitt 20 durch einen biegsamen Abschnitt 11 verbunden. Der biegsame Abschnitt 11 sorgt dafür, dass der Lordosenabschnitt 20 vom Rückenbereich 10 der Pelotte getrennt verschwenkbar ist. Dieser biegsame Abschnitt 11 kann dadurch gebildet sein, dass der Kunststoff, aus dem die Pelotte gefertigt ist, in diesem Bereich z. B. nach Art eines Filmscharniers ausgebildet ist. Es besteht allerdings auch die Möglichkeit, diesen biegsamen Abschnitt 11 aus Federstahl herzustellen, der beim Spritzen der Pelotte mit dem Kunststoff umspritzt wird.

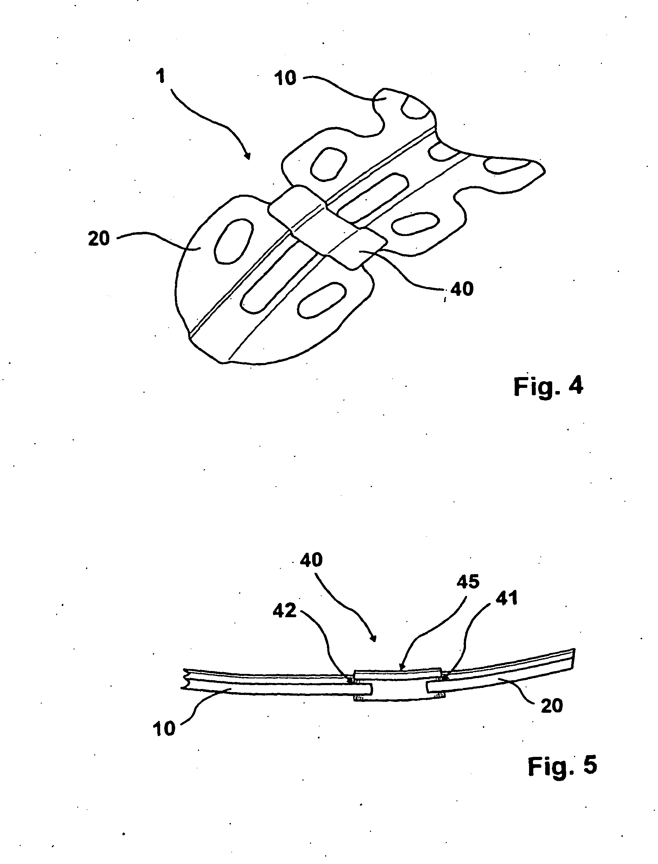

Nachfolgend wird die Pelotte gemäß der in den Fig. 4 und 5 dargestellten Ausführungsform beschrieben. Zur Verbindung des Rückenbereichs 10 der Pelotte 1 mit dem Lordosenabschnitt 20 ist ein Verbindungselement 40 vorgesehen. Das Verbindungselement 40 aus elastischem Material, z. B. TPE, weist seitlich zwei U-förmige Ausnehmungen 41, 42 zur Aufnahme der Enden der Pelottenteile auf. Im Bereich der Ausnehmung ist das Verbindungselement der Kontur der Pelotte und des Lordosenabschnitts nachempfunden, um eine größere Verbindungsfläche bereitzustellen. Der Mittelabschnitt 45 besteht aus Vollmaterial; das Verbindungselement, als gesondertes Teil, ist so elastisch, dass der Lordosenabschnitt 20 dauerhaft leicht schwenkbar relativ zur Pelotte bzw. des Rückenbereichs 10 der Pelotte 1 ist.

Die Pelotte, die zur Erhöhung des Komforts und zur Vermeidung von Druckstellen in Querrichtung gewölbt ausgebildet ist, besitzt mittig eine Wölbung, die wiederum zwei Abschnitte 2 und 12 bildet. Der Abschnitt 2 ist von dem Abschnitt 12 im Bereich des biegsamen Abschnittes 11 geteilt. Dies deshalb, um die relative Verschwenkbarkeit des Bereiches 10 der Pelotte im Lendenwirbelbereich gegenüber dem Rückenbereich 10 der Pelotte nicht einzuschränken.

Zu beiden Seiten der Wölbung oder Ausbuchtung 2, 12 erstreckt sich der Stützrand 3, 13. Im Bereich des Stützrandes 3 befinden sich beabstandet zueinander in Längsrichtung die Ausnehmungen 4, die in Verbindung mit der Ausbuchtung 2 dafür sorgen, dass die Pelotte um ihre Längsachse tordierbar ist. Dies vor dem Hintergrund, dass durch die Pelotte zwar eine Stabilisierung des Rückens in Längsrichtung erfolgen soll, die Beweglichkeit in Querrichtung, also die Verdrehung des Oberkörpers relativ zur Hüfte, nicht wesentlich eingeschränkt sein soll.

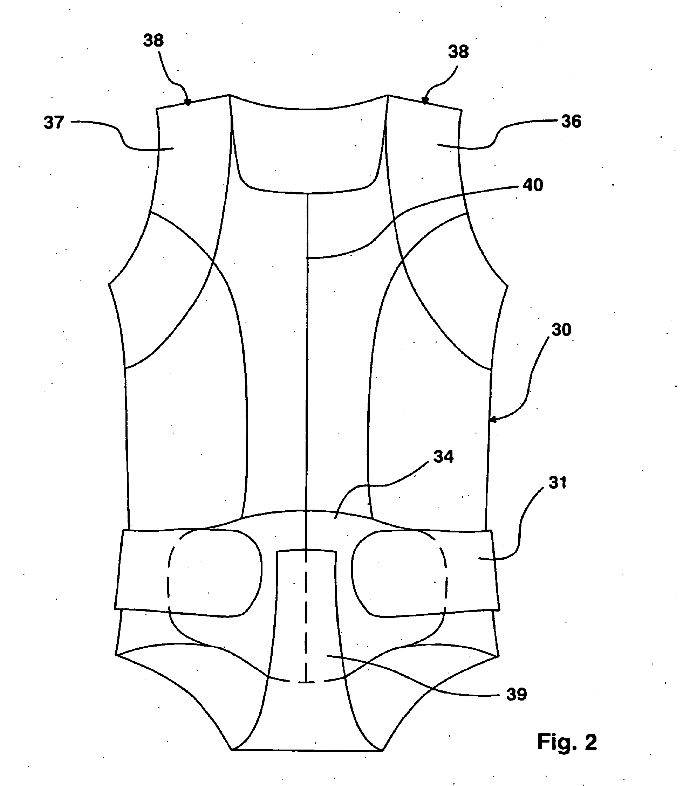

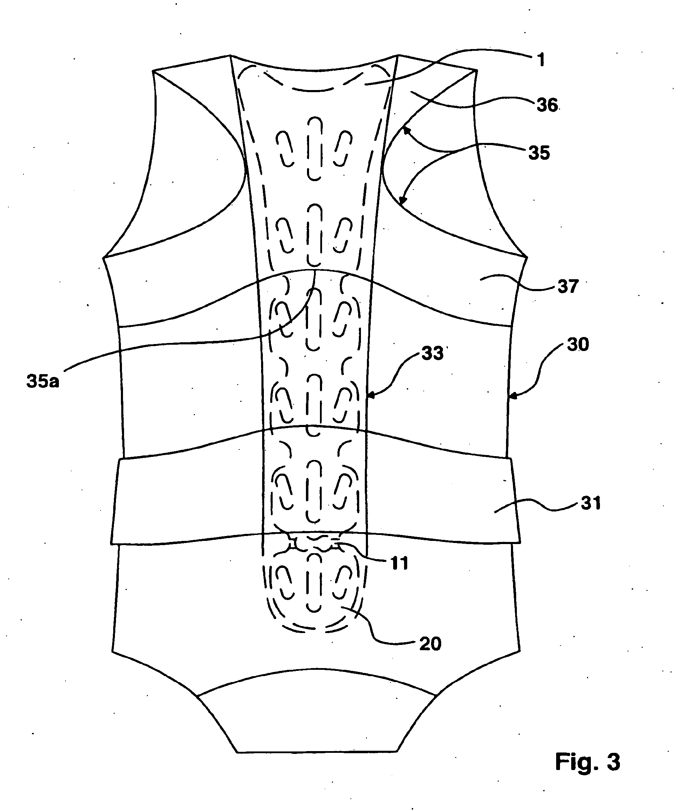

Der Stützrand 3 ist im Bereich des Nackens (Pfeil 5) breiter ausgebildet, als im unteren Bereich. Der Grund hierfür liegt darin, dass, wie bereits erläutert, in diesem Bereich die Pelotte die größtmögliche Kraft auf das Mieder bei nach vorne gebeugter Stellung des Rückens ausübt, und insofern eine große Fläche zur Verfügung stehen muss, um durch das Mieder den Patienten daran zu erinnern, den Rücken in eine aufrechte oder senkrechte Position zu bringen. Die Ausbuchtung 2, 12 dient schlussendlich auch dazu, dafür zu sorgen, dass die Pelotte nicht unmittelbar auf die Wirbel der Wirbelsäule drückt. Dies insbesondere vor dem Hintergrund, dass durch das Mieder (Fig. 2, Fig. 3) die Pelotte 1 im Bereich der Lendenwirbel oder Lordose (Pfeil 6) durch den Querzug 31 des Mieders 30 an den Rücken gepresst gehalten wird. Der Querzug 31 ist im Bereich der Rückentasche 33 für die Pelotte 1 an dem Mieder befestigt, z. B. durch einen Klettverschluss, wobei darauf hinzuweisen ist, dass der Querzug 31 die Pelotte oberhalb des biegsamen Abschnittes 11 der Pelotte kreuzt. Das heißt, dass beim Bücken die Pelotte am Rücken im Bereich der Lendenwirbel anliegt, wohingegen sich der Lendenwirbelbereich 20 der Pelotte relativ dazu bewegen kann, eben aufgrund des biegsamen Abschnittes 11.

Der Querzug 31 ist durch einen Klettbandverschluss 34 an dem Mieder vorne befestigbar. Durch den Klettbandverschluss wird erreicht, dass der Querzug an den Körperumfang anpassbar ist.

Im Schulterbereich befindet sich der mit insgesamt mit 35 bezeichnete Kreuzzug. Der Kreuzzug besteht aus zwei sich im Bereich der Einschubtasche 33 für die Pelotte 1 sich kreuzende Züge 36 und 37, wobei sich die Züge allerdings nicht über den Bereich der Einschubtasche 33 erstrecken, sondern lediglich an der Einschubtasche im Randbereich befestigt sind. Allerdings sind die Züge 36 und 37 durch eine einzige über die Einschubtasche geführte Naht 35a verbunden. Das betrifft die beiden unteren Nähte der Züge (Fig. 3); die übrigen Nähte der Kreuzzüge 36, 37 enden in der Naht der Einschubtasche. Im Brustbereich des Mieders gehen die Züge 36 und 37 in den Schultersteg 38 über. Das heißt, die kreuzweise Führung der Züge 36 und 37 findet nur im Rückenbereich statt. Durch die kreuzweise Führung der Züge 36 und 37 wird erreicht, dass die Kraft, die von der Pelotte auf das Mieder ausgeübt wird, wenn der Rücken eine gekrümmte Position einnimmt, diese Kraft auch von dem Mieder auf die Person übertragen wird, und insofern sich die Person daran erinnert, den Rücken gerade auszurichten.

Auf der Vorderseite des Mieders befindet sich ein Reißverschluss 40, der von beiden Enden her öffenbar ist, was der Steigerung des Komforts beim täglichen Tragen dient.

Die Schrittlasche 39 des Mieders ist ebenfalls mit einem Klettbandverschluss auf der Vorderseite des Mieders fixiert.

## Patentansprüche

1. Osteoporose-Orthese, umfassend eine Pelotte(s) und ein Mieder (30) mit einer Rückentasche (33) zur Aufnahme der Pelotte(s), wobei die Pelotte (1) um ihre Längsachse tordierbar ist,
**dadurch gekennzeichnet,**
**dass** die Pelotte (1) im Bereich der Lordose einen quer zur Längsachse biegsamen Abschnitt (11) aufweist, wobei der biegsame Abschnitt (11) durch ein von dem Material der Pelotte (1) unterschiedlichem Material gebildet ist.

2. Osteoporose-Orthese nach Anspruch 1,
**dadurch gekennzeichnet,**
**dass** die Pelotte (1) im Querschnitt im Bereich der Wirbelsäule eine sich in Längsrichtung erstreckende Ausbuchtung (2, 12) aufweist, wobei sich zu beiden Seiten der Ausbuchtung (2, 12) jeweils ein Stützrand (3, 13) erstreckt, mit dem die Pelotte (1) auf den Rippen aufliegt.

3. Osteoporose-Orthese nach einem der voranstehenden Ansprüche,
**dadurch gekennzeichnet,**
**dass** der biegsame Abschnitt (11) durch ein Verbindungselement (40) bereitgestellt wird, der die Pelotte mit einem Lordosenabschnitt (20) verbindet.

4. Osteoporose-Orthese nach einem der voranstehenden Ansprüche,
**dadurch gekennzeichnet,**
**dass** das Verbindungselement (40) zu jeder Seite eine quer zur Längsachse der Pelotte verlaufende U-förmige Aussparung (41, 42) aufweist.

5. Osteoporose-Orthese nach einem der voranstehenden Ansprüche,
**dadurch gekennzeichnet,**
**dass** das Verbindungselement (40) einen Mittelabschnitt (45) aufweist, der aus biegsamem Vollmaterial ausgebildet ist.

6. Osteoporose-Orthese nach einem der voranstehenden Ansprüche,
**dadurch gekennzeichnet,**
**dass** die Pelotte (1) in Querrichtung gewölbt ist.

7. Osteoporose-Orthese nach einem der voranstehenden Ansprüche,
**dadurch gekennzeichnet,**
**dass** der Stützrand (3, 13) im Nackenbereich (Pfeil 5) breiter ist als im Lordosenbereich.

8. Osteoporose-Orthese nach einem der voranstehenden Ansprüche,
**dadurch gekennzeichnet,**
**dass** der Stützrand (3, 13) zu jeder Seite beabstandet zueinander in Längsrichtung Einschnitte oder Ausnehmungen (4) aufweist.

9. Osteoporose-Orthese nach einem der voranstehenden Ansprüche,
**dadurch gekennzeichnet,**
**dass** die Pelotte (1) aus thermoplastischen Material besteht.

10. Osteoporose-Orthese nach einem der voranstehenden Ansprüche,
**dadurch gekennzeichnet,**
**dass** der Stützrand (3, 13) sich seitlich über die Muskelseitenstränge erstreckt.

## Claims

1. An osteoporosis orthosis, including a pad (1) and a corset (30) with a back pocket (33) for receiving said pad (1), said pad (1) being capable of torsion about its longitudinal axis,
**characterized in**
**that** said pad (1) comprises a portion (11) that is flexible transverse to the longitudinal axis in the region of the lordosis, said flexible portion (11) being made from a material that is different from that of said pad (1).

2. The osteoporosis orthosis as set forth in claim 1,
**characterized in**
**that** the pad (1) comprises in cross section, in the region of the spine, a bulge (2, 12) extending in the longitudinal direction, a supporting edge (3, 13) extending on either side of said bulge (2, 12) by which the pad (1) rests on the ribs.

3. The osteoporosis orthosis as set forth in any one of the previous claims,
**characterized in**
**that** the flexible portion (11) is provided by a connecting element (40) connecting the pad to a lordosis portion (20).

4. The osteoporosis orthosis as set forth in any one of the previous claims,
**characterized in**
**that** the connecting element (40) comprises on either side a U-shaped cutout (41, 42) extending transverse to the longitudinal axis of the pad.

5. The osteoporosis orthosis as set forth in any one of the previous claims,
**characterized in**
**that** the connecting element (40) comprises a central portion (45) that is made from a flexible solid material.

6. The osteoporosis orthosis as set forth in any one of the previous claims,
**characterized in**
**that** the pad (1) is curved in the transverse direction.

7. The osteoporosis orthosis as set forth in any one of the previous claims,
**characterized in**
**that** the supporting edge (3, 13) is wider in the nape region of the neck (arrow 5) than in the lordosis region.

8. The osteoporosis orthosis as set forth in any one of the previous claims,
**characterized in**
**that** the supporting edge (3, 13) comprises on either side notches or recesses (4) arranged in a spaced-apart relationship in the longitudinal direction.

9. The osteoporosis orthosis as set forth in any one of the previous claims,
**characterized in**
**that** the pad (1) is made from a thermoplastic material.

10. The osteoporosis orthosis as set forth in any one of the previous claims,
**characterized in**
**that** the supporting edge (3, 13) extends laterally over the erector spinae group.

## Revendications

1. Orthèse pour ostéoporose, du type comprenant une pelote (1) et un corset (30) avec une poche dorsale (33) destinée à recevoir la pelote (1), laquelle pelote (1) est mobile en torsion autour de son axe longitudinal,
**caractérisée en ce**
**que** la pelote (1) comporte à la hauteur de la lordose une partie (11) flexible transversalement par rapport à l'axe longitudinal, cette partie (11) flexible étant réalisée dans un matériau différent de celui de la pelote (1).

2. Orthèse pour ostéoporose selon la revendication 1,
**caractérisée en ce**
**que** la pelote (1) comporte, en section transversale à la hauteur de la colonne vertébrale, un renflement (2, 12) s'étendant suivant la direction longitudinale, un bord de support (3, 13) par lequel la pelote (1) repose sur les côtes s'étendant de part et d'autre du renflement (2, 12).

3. Orthèse pour ostéoporose selon l'une quelconque des revendications précédentes,
**caractérisée en ce**
**que** la partie (11) flexible est fournie par un élément de liaison (40) reliant la pelote à une partie lordotique (20).

4. Orthèse pour ostéoporose selon l'une quelconque des revendications précédentes,
**caractérisée en ce**
**que** l'élément de liaison (40) comporte de chaque côté une découpe (41, 42) en U s'étendant transversalement par rapport à l'axe longitudinal de la pelote.

5. Orthèse pour ostéoporose selon l'une quelconque des revendications précédentes,
**caractérisée en ce**
**que** l'élément de liaison (40) comporte une partie centrale (45) qui est réalisée en un matériau plein flexible.

6. Orthèse pour ostéoporose selon l'une quelconque des revendications précédentes,
**caractérisée en ce**
**que** la pelote (1) est bombée transversalement.

7. Orthèse pour ostéoporose selon l'une quelconque des revendications précédentes,
**caractérisée en ce**
**que** le bord de support (3, 13) est plus large à la hauteur de la nuque (flèche 5) qu'à la hauteur de la lordose.

8. Orthèse pour ostéoporose selon l'une quelconque des revendications précédentes,
**caractérisée en ce**
**que** le bord de support (3, 13) comporte de chaque côté des encoches ou des évidements (4) disposés avec un écartement entre eux dans le sens de la longueur.

9. Orthèse pour ostéoporose selon l'une quelconque des revendications précédentes,
**caractérisée en ce**
**que** la pelote (1) est réalisée en un matériau thermoplastique.

10. Orthèse pour ostéoporose selon l'une quelconque des revendications précédentes,
**caractérisée en ce**
**que** le bord de support (3, 13) s'étend latéralement sur les muscles paravertébraux.
